# EUROPÄISCHE PATENTSCHRIFT

(11) **EP 0 359 042 B1**
(45) Veröffentlichungstag und Bekanntmachung des Hinweises auf die Patenterteilung: **01.02.1995**
(21) Anmeldenummer: 89116069.9
(22) Anmeldetag: 31.08.1989
(51) Int. Cl.: C07H 7/027

(54) **Verfahren zur Isolierung von 2-Keto-polyhydroxy-C6-carbonsäuren, insbesondere von 2-Keto-L-gulonsäure aus wässrigen Fermentationsausträgen**
Process for the isolation of 2-keto-polyhydroxy-C6-carboxylic acids, especially 2-keto-L-gulonic acid, from aqueous fermentation media
Procédé d'isolation d'acides 2-kéto-polyhydroxy-C6-carboxyliques, notamment l'acide 2-kéto-L-gulonique, d'un bouillon de fermentation aqueux

(30) Priorität: 13.09.1988 DE 3831070
(43) Veröffentlichungstag der Anmeldung: 21.03.1990
(73) Patentinhaber: BASF Aktiengesellschaft, 67063 Ludwigshafen (DE)
(72) Erfinder: Schaper, Michael, Dr., D-6700 Ludwigshafen (DE)

(56) Entgegenhaltungen:
- EP-A- 0 086 324
- EP-A- 0 178 493
- CHEMICAL ABSTRACTS, Band 87, Nr. 23, 5. Dezember 1977, Seite 462,Zusammenfassung Nr. 182601y, Columbus, Ohio, US;
- & JP-A-77 66 684 (SHIONOGI AND CO., LTD) 02-06-1977 (Kat. D)

## Beschreibung

Die vorliegende Erfindung betrifft die Isolierung von auf fermentativem Wege hergestellten 2-Keto-polyhydroxy-C₆-carbonsäuren, insbesondere von so hergestellter 2-Keto-L-gulonsäure aus wäßrigen Fermentationsausträgen mittels Extraktion in Gegenwart von Aminen.

### Stand der Technik:

2-Keto-L-gulonsäure ist ein zentrales Zwischenprodukt bei der Herstellung von Vitamin C. Vitamin C wurde bisher weltweit konventionell nach dem sogenannten Reichsteinverfahren hergestellt. Bei diesem Verfahren handelt es sich um einen vielstufigen Prozeß, der vor allem aufgrund der aufwendigen Schutzgruppenchemie nicht mehr zeitgemäß ist.

Aus diesem Grunde hat es nicht an Versuchen gefehlt, Verfahren zur Herstellung des begehrten Vitamin C auf biotechnologischer Basis zu entwickeln, die weniger Reaktionsschritte aufweisen und bei denen die Problematik geeigneter Schutzgruppen vermieden wird. Allen bisher bekannten wichtigen biotechnologischen Verfahren gemeinsam ist die Verwendung von D-Glucose als Ausgangsverbindung und die Bildung von 2-Keto-L-gluconsäure (2-KGS) als Vitamin-C-Vorstufe. Bekannte biotechnologische Wege:
a) Glucose → 2,5-Diketogluconsäure → 2-KGS → Vitamin C
b) Glucose → L-Sorbose → 2-KGS → Vitamin C.

Da die 2-KGS in beiden Fällen durch Fermentation erzeugt wird, findet man sie in wäßriger Lösung zusammen mit Biomasse vor. Für die Gewinnung reiner L-Ascorbinsäure (Vitamin C) ist aber eine Isolierung der 2-KGS bzw. eine Isolierung von deren Derivaten aus den Fermentationsausträgen unumgänglich.

Bei den meisten bisher bekannten fermentativen Verfahren wird der für die Fermentation geeignete pH-Wert durch Zufügen eines Calciumsalzes eingestellt, wodurch 2-KGS in Form ihres Calciumsalzes in der wäßrigen Fermentationsbrühe anfällt.

Nach Shionogi (vgl. JA-OS 52 066684) wird aus dieser Lösung das Calcium durch Zugabe von Natriumcarbonat als Calciumcarbonat gefällt und abfiltriert, das Filtrat eingedampft und durch Zusatz von Methanol 2-KGS als Natriumsalz ausgefällt. Die Ausbeute beträgt hierbei ca. 87 %. Durch Behandeln des Natriumsalzes mit HCl in einem Lösungsmittel werden nacheinander NaCl und Vitamin C erhalten (vgl. EP-A 91 134). Die Umlagerung verläuft mit 72 % Ausbeute.

Gemäß Verfahren von Takeda (vgl. JA-AS 75 022 113) erfolgt die Isolierung von 2-KGS durch Ionenaustausch. Nach Veresterung der Säure wird mit Natriummethylat zum Natriumascorbat in 86%iger Ausbeute umgelagert.

Nachteilig bei allen diesen Verfahren ist, daß große Fermentationsvolumina bewegt werden müssen und daß Natriumketogulonat mit HCl zur Ascorbinsäure sauer umgelagert wird, wobei eine große Menge Satz als unerwünschtes Nebenprodukt entsteht. Entsprechend wird bei der alkalischen Umlagerung des Esters Natriumascorbat erzeugt dessen Überführung in L-Ascorbinsäure ebenfalls die Bildung großer Mengen Satz mit sich bringt, was aus umwelttechnischen und Kostengründen unerwünscht ist.

Es war daher die Aufgabe der Erfindung, ein möglichst einfaches Verfahren zur Isolierung von 2-KGS aus wäßrigen Fermentationsausträgen zu entwickeln, bei dem die Nachteile des Standes der Technik nicht auftreten, bei dem insbesondere die Bildung großer Mengen an Salzen entfällt.

Es wurde nun überraschenderweise gefunden, daß 2-KGS in Gegenwart von Aminen mit 15 bis 40 C-Atomen unter ganz bestimmten Bedingungen in einfacher Weise durch Extraktion mit höheren Alkanolen aus der Fermentationsbrühe abgetrennt werden und aus dem gebildeten 2-KGS-Amin-Addukt auf einfache Weise als 2-KGS, 2-KGS-Salz oder 2-KGS-Ester gewonnen werden kann.

Die Extraktion organischer Säuren in Gegenwart von Aminen durch Ionenpaar-Bildung ist zwar prinzipiell bekannt (vgl. Analytica Chimica Acta 165 [1984] Seiten 245-256 und DE-A 34 36 348), bei den bekannten Verfahren zur Extraktion von organischen Säuren aus wäßrigen Lösungen in Gegenwart von Aminen handelt es sich aber meist um Verfahren zur Extraktion von einfachen niederen Carbonsäuren wie Milchsäure, Buttersäure, Äpfelsäure und Citronensäure.

Dazu werden Gemische aus einem langkettigen Amin, insbesondere Trilaurylamin, einem mit Wasser nicht oder nur schlecht mischbaren Solvens sowie Zusätzen aus organischen Säuren, wie Ölsäure oder Diethylhexylphosphorsäure bereitet und mit der zu extrahierenden wäßrigen säureenthaltenden Lösung in Kontakt gebracht (vgl. EP 49 429).

Gemäß dem Verfahren von DE-A 34 36 348 wird als Extraktionsmittel ein aus einem längerkettigen aliphatischen Amin und einem Phenol bzw. Naphthol bestehendes Gemisch zur Extraktion einer wäßrigen Carbonsäurelösung mit einem Gehalt < 8 Gew.-% eingesetzt.

Die Extraktion niederer Fettsäuren aus wäßrigen Lösungen mittels einem Gemisch aus einem tert.-Amin und einem verzweigten primären Alkohol wurde in JA-OS 61-176 552 bzw. JA-OS 61-176 553; beschrieben.

Die Anwendung der bekannten Extraktionsbedingungen auf die Gewinnung von 2-KGS aus Fermentationsausträgen lieferte keine oder nur sehr schlechte, wirtschaftlich unakzeptable Ausbeuten an 2-KGS.

Dies dürfte einerseits in dem üblicherweise zu starker Emulsionsbildung führenden stark polaren Charakter von 2-KGS 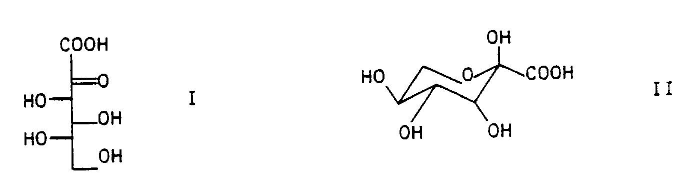
begründet sein (vgl. Polyhydroxyketocarbonsäure I), andererseits dadurch, daß 2-KGS aufgrund der Oxo-Cyclo-Tautomerie (vgl. II mit zuckerähnlicher Struktur und zuckerähnlichem Verhalten) zahlreiche Nebenreaktionen eingehen kann.

Überraschenderweise ist eine Gewinnung der 2-KGS mit Aminen aus Fermentationsbrühe durch Wahl spezieller Bedingungen in sehr guten Ausbeuten möglich, und daher für die technische Nutzung geeignet.

Gegenstand der Erfindung ist daher ein Verfahren zur Extraktion von 2-Keto-polyhydroxy-C₆-carbonsäuren, insbesondere von 2-KGS, aus wäßrigen Fermentationsausträgen, das dadurch gekennzeichnet ist, daß man
a) aus einem von Biomasse befreiten Fermentationsaustrag die enthaltenen Calciumionen in an sich bekannter Weise entfernt,
b) den calciumionenfreien Fermentationsaustrag in Gegenwart von einem Amin mit insgesamt 15 bis 40 C-Atomen in einer Menge von 0,7 bis 2,5 Molequivalenten, bezogen auf die in dem Fermentationsaustrag enthaltene 2-Keto-polyhydroxy-C₆-carbonsäure, mit einem C₄- bis C₈-Alkanol in einer Menge von 1,5 bis 8, vorzugsweise 2 bis 6, insbesondere 2,5 bis 5 Gew.-Teilen, bezogen auf das Amin, bei Temperaturen zwischen 10 und 35°C, vorzugsweise 20 bis 25°C, extrahiert und
c) das erhaltene 2-Keto-polyhydroxy-C₆-carbonsäure-Addukt in die freie Carbonsäure oder deren Derivat spaltet bzw. überführt.

Als 2-Keto-polyhydroxy-C₆-carbonsäure, die erfindungsgemäß aus sie enthaltenden Fermentationsausträgen isoliert werden können, kommen insbesondere 2-Keto-L-gulonsäure, 2-Keto-D-gluconsäure und 2-Keto-galaktonsäure in Betracht. Von besonderer Bedeutung ist das Verfahren für die Isolierung der für die Herstellung von Vitamin C dringend benötigten 2-Keto-L-gulonsäure. Die Ketocarbonsäuren fallen bei ihrer fermentativen Herstellung im allgemeinen in Fermentationsbrühen an, die zwischen 1 und 20 Gew.-%, vorzugsweise etwa 7 - 12 Gew.-% der Carbonsäure enthalten.

Vor dem Extraktionsprozeß wird aus diesen Fermentationsbrühen die Biomasse in an sich bekannter Weise durch Abfiltrieren oder Zentrifugieren abgetrennt.

Um eine effiziente 2-KGS-Extraktion zu erreichen, ist es weiterhin zwingend erforderlich, von einem absolut metallionenfreien Fermentationsaustrag auszugehen. Anderenfalls hemmt das 2-KGS-Metalion-Ionenpaar die Bildung des extrahierbaren 2-KGS-Amin-Adduktes. Die Abtrennung der zumeist enthaltenen Calciumionen aus dem Fermentationsaustrag erfolgt in an sich bekannter Weise. Mit besonderem Vorteil arbeitet man so, daß man zunächst mit verdünnter Schwefelsäure enthaltene Calciumionen als Calciumsulfat ausfällt und nach Abfiltrieren des gebildeten Calciumsulfats das Filtrat mit einem Kationenaustauscher behandelt, vorzugsweise durch eine Kationenaustauschersäule leitet.

Zur Extraktion werden dem Fermentationsaustrag etwa 0,7 bis 2,5 vorzugsweise 1 bis 1,5 Molequivalente, insbesondere etwa 1,0 bis 1,2 Molequivalente, bezogen auf die in dem Fermentationsaustrag enthaltene 2-Keto-polyhydroxy-C₆-carbonsäure, des Amins zugefügt. Als Amine kommen langkettige, lipophile Amine mit aliphatischen, cycloaliphatischen und aromatischen Resten mit insgesamt 15 bis 40 C-Atomen in Betracht. Genannt seien insbesondere Trioctylamin, Trilaurylamin, Tri-tridecylamin, Tricaprylamin, käufliche Gemische aus Trilauryl- und Dilaurylamin (Tridodecylamin) und Ditridecylamin.

Mit besonderem Vorteil arbeitet man mit handelsüblichem Trioctylamin, Tridodecylamin oder Ditridecylamin.

Als eigentliche Extraktionsmittel dienen C₄- bis C₈-Alkanole, wie n-Butanol, iso-Butanol, Pentanole, Hexanole, Heptanole und Octanole, insbesondere Methylisobutylcarbinol, n-Butanol und iso-Butanol. Die Alkanole verwendet man in Mengen von 1,5 bis 8, vorzugsweise 2 bis 6, insbesondere etwa 2,5 bis 5 Gew.-Teilen, bezogen auf das lipophile Amin. Die verwendete Gesamtmenge an langkettigem Amin und Alkanol richtet sich im allgemeinen nach der Zusammensetzung des Fermentationsaustrages. Durch einfache Vorversuche kann für jeden Fall festgestellt werden, bei welcher Menge eine gute Trennung erzielt werden kann und keine Emulsionsbildung auftritt. Größenordnungsmäßig arbeitet man mit einem Verhältnis von Fermenteraustrag zu Extraktionsgemisch von etwa 0,9/1 bis 1,2/1.

Die Extraktion kann im Chargen-(batch)-Betrieb, aber auch kontinuierlich (z.B. in einem Mixer-Settler) durchgeführt werden.

Beim Arbeiten im Chargen-Betrieb wird das Extraktionsgemisch etwa 15 bis 60 Minuten (min.), vorzugsweise etwa 30 min, bei einer Temperatur zwischen 10 und 35°C, vorzugsweise bei etwa 20 bis 25°C, gerührt.

Nach der Phasentrennung wird aus der das Wertprodukt als Amin-Addukt enthaltenden alkoholischen Phase unter vermindertem Druck der Alkohol destillativ entfernt. Bei Verwendung von Butanolen wird das 2-KGS-Amin-Addukt beispielsweise aus der Oberphase durch Abdestillieren des Alkohols bei etwa 10 bis 100 mbar, vorzugsweise 15 bis 25 mbar, mit Hilfe eines Rotationsverdampfers bei 20 bis 50°C, vorzugsweise etwa 30°C isoliert. Der abdestillierte Alkohol kann erneut zur Extraktion verwendet werden.

Die Unterphase kann einer weiteren Extraktion zugeführt werden. So kann durch 2-malige Extraktion beispielsweise die 2-Keto-L-gulonsäure als 2-KGS-Amin-Addukt in einer Gesamtausbeute von 99 % aus dem Fermentationsaustrag extrahiert werden.

Demgegenüber wird bei Einsatz einer Calciumionen enthaltenden Fermentationsbrühe auch bei Anwendung einer 5-stufigen Mixer-Settler-Einheit (mit 2,2 Moläquivalenten Amin pro Säure) eine Extraktionsausbeute von nur 80 bis 90 % erhalten.

Das bei der beschriebenen erfindungsgemäßen Extraktion anfallende 2-Keto-polyhydroxy-C₆-Carbonsäure-Amin-Addukt kann auf verschiedene Weise weiterverarbeitet werden. Der Einfachheit halber beschreiben wir im einzelnen die Weiterverarbeitung der besonders wichtigen 2-KGS.
1. Zur Gewinnung von freier 2-Keto-L-gulonsäure kann man das 2-KGS-Amin-Addukt mit etwa 3 bis 8 Gew.-Teilen Test-benzin (Kp 155 bis 185°C), bezogen auf das 2-KGS-Amin-Addukt, versehen und bei Temperaturen von 60 bis 120°C, vorzugsweise etwa 90°C, im Reaktionsgefäß für 10 bis 60 Minuten (min.), vorzugsweise etwa 30 min. rühren. Die ausfallenden 2-KGS-Kristalle werden heiß abgesaugt. Man erzielt hierbei Ausbeuten von etwa 94 %. Das Amin kann in den Prozeß zurückgeführt werden.
   Das 2-KGS-Amin-Addukt kann aber auch mit Wasser bei Raumtemperatur (RT) in die freie 2-KGS und Amin gespalten werden. Hierzu wird das erhaltene 2-KGS-Amin-Addukt durch Behandeln mit 2 bis 20, vorzugsweise etwa 10 Gew.-Teilen Wasser, bezogen auf das Addukt, bei Temperaturen von 5 bis 50°C, vorzugsweise etwa 20°C in die freie 2-KGS und das eingesetzte Amin gespalten, wozu im allgemeinen Reaktionszeiten von etwa 10 bis 60 Minuten notwendig sind.
2. Zur Gewinnung von Alkalisalzen der 2-KGS wird das 2-KGS-Amin-Addukt im allgemeinen mit der 2- bis 5-fachen, vorzugsweise etwa 3fachen Gewichtsmenge an Wasser versetzt und das erhaltene Gemisch anschließend mit einer Alkalihydroxidlösung titriert bis ein pH-Wert zwischen 9 und 12, vorzugsweise etwa 10 bis 11 erreicht ist. Das Amin kann aus dem erhaltenen Gemisch beispielsweise mit Hexan extrahiert und nach destillativem Entfernen des Hexans erneut verwendet werden. Aus der wäßrigen Phase kann das Alkalisalz der 2-KGS durch Eindampfen in etwa 97%iger Ausbeute erhalten werden.
   Auf analoge Weise können durch Umsetzen mit Erdalkalihydroxiden die entsprechenden Erdalkalisalze der 2-KGS erhalten werden.
3. Zur Gewinnung von 2-KGS-Estern wird das 2-KGS-Amin-Addukt mit der 1,5-bis 4-fachen Gewichtsmenge einer etwa 2,5 N Lösung HCl in einem C₁- bis C₅-Alkanol, vorzugsweise in Methanol und einem inerten Lösungsmittel, wie Methylenchlorid versetzt und mehrere Stunden (h), vorzugsweise etwa 12 h bei RT gerührt. Hierbei kristallisiert der sich bildende Ester aus dem Reaktionsgemisch aus und kann durch Filtration abgetrennt werden. Die Ausbeute an 2-KGS-methylester beträgt bei diesem Verfahren etwa 95,9 % der Theorie.

Mit Hilfe des erfindungsgemäßen Verfahrens können 2-Keto-polyhydroxy-C₆-carbonsäuren, insbesondere die für die Herstellung von Vitamin C sehr begehrte 2-Keto-L-gulonsäure, auf relativ einfache und wirtschaftlich vorteilhafte Weise aus wäßrigen Fermentationsausträgen in reiner Form oder in Form von ihren Salzen oder Estern isoliert werden. Die so erhaltene 2-KGS bzw. deren Derivate weisen eine hohe Reinheit auf und können auf bekannte Weise in Vitamin C überführt werden.

Die folgenden Beispiele sollen das erfindungsgemäße Verfahren erläutern.

### Beispiel 1

### A. Entfernen von Calciumionen

2400 g eines mikrofiltrierten, klaren Fermentationsaustrages (2-KGS-Gehalt nach HPLC 6,7 %) wurden auf 50°C erwärmt und mit 250 ml einer 20 gew.-%-igen wäßrigen Schwefelsäure auf einen pH-Wert von 1,3 bis 1,4 eingestellt. Anschließend wurde das Gemisch 30 min. gerührt, auf Raumtemperatur (RT) abgekühlt und dann das ausgefallene CaSO₄ (59 g) abgesaugt. Das erhaltene Filtrat wurde durch eine Kationenaustauschersäule (⌀ 4 cm; Füllhohe 40 cm) gefüllt mit Lewatit S 100 G1 laufen lassen. Das Eluat enthielt (nach einem Test mit Metall-indikator-Puffer-Tabletten) kein Calcium mehr. Es wurde unter vermindertem Druck eingeengt.

### B. Extraktion von 2-KGS in Gegenwart von Trioctylamin

300 g eines biomasse- und metallionenfreien 2-KGS-Fermentationsaustrages (lt. HPLC 10,1%ig), der 30,3 g (entsprechend 0,156 Mol) 2-KGS enthielt, wurde mit 210 g n-Butanol und 66 g (0,186 Mol, entsprechend 1,18 Molequivalenten, bezogen auf 2-KGS) Trioctylamin versetzt und im Scheidetrichter 30 min. bei RT gerührt. Nach Phasentrennung wurden 175 g einer hellgelben klaren Unterphase erhalten, die nach HPLC noch 2 g 2-KGS enthielt. Das entspricht einer Extraktion von 93,4 %. Die Oberphase (399,5 g) wurde an Rotationsverdampfer unter stark vermindertem Druck eingeengt. Man erhielt 294 g eines Destillates, das aus 190 g n-Butanol und 104 g Wasser bestand. Es verblieben 101,2 g Rückstand in Form einer klaren, braunen viskosen Masse.

### B. Thermische Spaltung des 2-KGS-Trioctylamin-Adduktes

Die gemäß A erhaltenen 101,2 g des 2-KGS-Trioctylamin-Adduktes, enthaltend 28,3 g 2-KGS, wurden in 800 ml Testbenzin (Kp. 155 bis 185°C) gelöst und bei Innentemperaturen von 90°C etwa 30 min. gerührt.

Die ausgefallenen 2-KGS-Kristalle wurden heiß abgesaugt, mit CH₂Cl₂ nachgewaschen bis ein farbloses Filtrat erhalten wurde und dann trockengesaugt. Man erhielt 27,3 g an beigefarbenen Kristallen, die nach NaOH-Titration zu 97 % aus 2-KGS bestanden und einen Wassergehalt von etwa 1,5 bis 2 % aufwiesen. Die Ausbeute betrug somit 93,6 % bezogen auf eingesetztes Amin-Addukt.

Die hierbei angefallene Mutterlauge wurde mit ca. 50 ml Wasser versetzt, mit 40 ml 1N NaOH auf pH 10,3 gestellt und die Phasen voneinander getrennt.

Die wäßrige Phase enthielt ca. 1 bis 1,5 g 2-KGS. Nach Einengen derselben verblieben 7,0 g eines dunkelbraunen Feststoffes. Die organische Phase wurde unter stark vermindertem Druck bei Badtemperaturen von 100°C von Lösungsmittel befreit. Hierbei verblieben als Rückstand 68,5 g eines gelbbraunen Öls, das nach gaschromatographischer Untersuchung (GC) zu etwa 90 % aus Trioctylamin und zu etwa 10 % aus Lösungsmittel bestand.

### Beispiel 2

### A. Extraktion mit iso-Butanol in Gegenwart von Trioctylamin

300 g eines von Biomasse und Metallionen befreiten 2-KGS-Fermentationsaustrages, enthaltend 30,6 g, entsprechend 157,7 mMol 2-KGS, wurden mit 214 ml iso-Butanol und 66 g (186,6 mMol, entsprechend 1,18 Molequivalenten, bezogen auf 2-KGS) Trioctylamin versetzt und das Gemisch 30 min. bei RT gerührt. Nach 30 min. erhielt man eine klare Phasentrennung. Die Unterphase (175 g) enthielt nach Einengen 1,75 g 2-KGS. Die Oberphase (404 g) ergaben nach Einengen unter stark vermindertem Druck einen Rückstand von 104,5 g 2-KGS-Trioctylamin-Addukt. Das entspricht rechnerisch einer Extraktion von 94,3 %.

### B. Alkalische Spaltung des 2-KGS-Trioctylamin-Adduktes

10 g des gemäß 2A erhaltenen Rückstandes, enthaltend 2,75 g = 14,2 mMol 2-KGS, wurden mit 30 ml Wasser und 18 ml einer 1N-NaOH auf einen pH-Wert von 10,2 eingestellt. Das abgespaltene Amin wurde mit n-Hexan extrahiert. Die wäßrige Phase wurde eingeengt. Es verblieben 3,81 g eines braunen Feststoffes, enthaltend 2,97 g Na-2-KGS (100 %). Das entspricht einer Ausbeute von etwa 97 %.

### C. Esterspaltung

10 g des gemäß 2A erhaltenen Rückstandes wurden mit 20 ml einer 2,5 N Lösung von HCl in Methanol und 30 ml CH₂Cl₂ versetzt. Man erhielt eine klare Lösung, die über Nacht gerührt wurde. Anschließend wurde der ausgefallene Ester abgesaugt, mit CH₂Cl₂ nachgewaschen und trockengesaugt. Man erhielt 2,53 g eines cremefarbenen Feststoffs, der nach HPLC eine Reinheit von 97 % aufwies, was einer Ausbeute an 83,4 % entspricht.

Aus der Mutterlauge wurden bei -20°C noch einmal 0,4 g eines Festoffs erhalten, der nach HPLC eine Reinheit von 91 % aufwies, entsprechend einer Ausbeute von 12,4 %.

Das ergibt eine Gesamtausbeute von 95,8 %.

### D. Spaltung in Wasser

10 g des gemäß 2A erhaltenen Rückstandes wurden mit 100 ml Wasser versetzt und 1 h bei RT gerührt. Anschließend wurde mit 40 ml n-Hexan und dann mit 40 ml CH₂Cl₂ extrahiert. Die wäßrige Phase enthielt 2,72 g 2-KGS. Nach dem Einengen unter vermindertem Druck wurden 3,2 g eines Feststoffs erhalten, der nach HPLC zu 82,2 % aus 2-KGS bestand. Das entspricht einer Ausbeute von 95,6 %.

## Patentansprüche

1. Verfahren zur Extraktion von 2-Keto-polyhydroxy-C₆-carbonsäuren aus wäßrigen Fermentationsausträgen, dadurch gekennzeichnet daß man
a) aus einem von der Biomasse befreiten Fermentationsaustrag die enthaltenen Calciumionen in an sich bekannter Weise entfernt,
b) den calciumionenfreien Fermentationsaustrag in Gegenwart von einem Amin mit insgesamt 15 bis 40 C-Atomen in einer Menge von 0,7 bis 2,5 Molequivalenten, bezogen auf die in dem Fermentationsaustrag enthaltene 2-Keto-polyhydroxy-C₆-carbonsäure, mit einem C₄- bis C₈-Alkanol in einer Menge von 1,5 bis 8 Gew.-Teilen, bezogen auf das Amin, bei Temperaturen zwischen 10 und 35°C extrahiert und
c) das erhaltene 2-Keto-polyhydroxy-C₆-carbonsäure-Amin-Addukt in die freie Carbonsäure oder deren Derivat spaltet bzw. überführt.

2. Verfahren gemäß Anspruch 1, dadurch gekennzeichnet, daß man einen 2-Keto-L-gulonsäure enthaltenden Fermentationsaustrag einsetzt.

3. Verfahren gemäß Anspruch 2, dadurch gekennzeichnet, daß man den calciumionenfreien Fermentationsaustrag in Gegenwart von 1 bis 1,2 Molequivalenten, bezogen auf die in dem Fermentationsansatz enthaltene 2-Keto-L-gulonsäure, des langkettigen Amins extrahiert.

4. Verfahren gemäß Anspruch 2, dadurch gekennzeichnet, daß man den von Calciumionen befreiten Fermentationsaustrag mit etwa 2 bis 6 Gew.-Teilen eines C₄- bis C₈-Alkanols, bezogen auf das Amin extrahiert.

5. Verfahren gemäß Anspruch 1, dadurch gekennzeichnet, daß man als Amin Trioctylamin, Tridodecylamin oder Ditridecylamin verwendet.

6. Verfahren gemäß Anspruch 1, dadurch gekennzeichnet, daß man als C₄- bis C₈-Alkanol ein Butanol verwendet.

7. Verfahren gemäß Anspruch 2, dadurch gekennzeichnet, daß man das erhaltene 2-Keto-L-gulonsäure-Amin-Addukt durch Erhitzen auf 60 bis 120°C in freie 2-Keto-L-gulonsäure und das eingesetzte Amin zurückspaltet.

8. Verfahren gemäß Anspruch 2, dadurch gekennzeichnet, daß man das erhaltene 2-Keto-L-gulonsäure-Amin-Addukt durch Behandeln mit 2 bis 20 Gew.-Teilen Wasser, bezogen auf das Addukt, bei Temperaturen von 5 bis 50°C, in die freie 2-Keto-L-gulonsäure und das eingesetzte Amin spaltet.

9. Verfahren gemäß Anspruch 2, dadurch gekennzeichnet, daß man das erhaltene 2-Keto-L-gulonsäure-Amin-Addukt mit einem Alkali- oder Erdalkalihydroxid in das entsprechende Salz der 2-Keto-L-gulonsäure überführt.

10. Verfahren gemäß Anspruch 2, dadurch gekennzeichnet, daß man das erhaltene 2-Keto-L-gulonsäure-Amin-Addukt mit einem Chlorwasserstoffgas enthaltendem C₁- bis C₅-Alkanol, ggf. in einem inerten Lösungsmittel, in den entsprechenden Alkanolester der 2-Keto-L-gulonsäure überführt.

## Claims

1. A process for extracting 2-keto-polyhydroxy-C₆-carboxylic acids from aqueous fermentation product mixtures, wherein
a) the calcium ions contained in a fermentation product mixture which has been freed from biomass are removed in a conventional manner,
b) the fermentation product mixture, free from calcium ions, is extracted in the presence of an amine of a total of 15 to 40 carbon atoms in an amount of from 0.7 to 2.5 mole equivalents, based on the 2-keto-polyhydroxy-C₆-carboxylic acid contained in the fermentation product mixture, by means of a C₄-C₈-alkanol in an amount of from 1.5 to 8 parts by weight, based on the amine, at from 10 to 35°C, and
c) the 2-keto-polyhydroxy-C₆-carboxylic acid amine adduct obtained is cleaved to give the free carboxylic acid or converted to a derivative thereof.

2. A process as claimed in claim 1, wherein a fermentation product mixture containing 2-keto-L-gulonic acid is employed.

3. A process as claimed in claim 2, wherein the fermentation product mixture, free from calcium ions, is extracted in the presence of from 1 to 1.2 mole equivalents, based on the 2-keto-L-gulonic acid contained in the fermentation batch, of the long-chain amine.

4. A process as claimed in claim 2, wherein the fermentation product mixture which has been freed from calcium ions is extracted with from about 2 to 6 parts by weight, based on the amine, of a C₄-C₈-alkanol.

5. A process as claimed in claim 1, wherein trioctylamine, tridodecylamine or ditridecylamine is used as the amine.

6. A process as claimed in claim 1, wherein a butanol is used as the C₄-C₈-alkanol.

7. A process as claimed in claim 2, wherein the 2-keto-L-gulonic acid amine adduct obtained is cleaved again to give the free 2-keto-L-gulonic acid and the amine employed by heating at from 60 to 120°C.

8. A process as claimed in claim 2, wherein the 2-keto-L-gulonic acid amine adduct obtained is cleaved to give the free 2-keto-L-gulonic acid and the amine employed by treatment with from 2 to 20 parts by weight of water, per part by weight of adduct, at from 5 to 50°C.

9. A process as claimed in claim 2, wherein the 2-keto-L-gulonic acid amine adduct obtained is converted by means of an alkali metal hydroxide or alkaline earth metal hydroxide into the corresponding salt of 2-keto-L-gulonic acid.

10. A process as claimed in claim 2, wherein the 2-keto-L-gulonic acid amine adduct obtained is converted, by means of a C₁-C₅-alkanol containing hydrogen chloride gas, if appropriate in an inert solvent, into the corresponding alkanol ester of 2-keto-L-gulonic acid.

## Revendications

1. Procédé pour l'extraction d'acides 2-céto-polyhydroxy-C6-carboxyliques à partir de charges aqueuses de fermentation, caractérisé
a) en ce qu'on enlève les ions calcium contenus d'une manière connue en soi d'une charge de fermentation libérée de la biomasse,
b) en ce qu'on extrait la charge de fermentation exempte d'ions calcium en présence d'une amine avec au total 15 à 40 atomes de carbone dans une quantité de 0,7 à 2,5 équivalents molaires par rapport à l'acide 2-céto-polyhydroxy-C6-carboxylique contenu dans la charge de fermentation, avec un alcanol en C₄ - C₈ dans une quantité de 1,5 à 8 parties en poids par rapport à l'amine, à des températures comprises entre 10 et 35°C et
c) et en ce qu'on coupe le produit d'addition acide 2-céto-polyhydroxy-C6-carboxylique/amine obtenu en acide carboxylique libre ou en son dérivé.

2. Procédé selon la revendication 1, caractérisé en ce qu'on utilise une charge de fermentation contenant de l'acide 2-céto-L-gulonique.

3. Procédé selon la revendication 2, caractérisé en ce qu'on extrait la charge de fermentation exempte d'ions calcium en présence de 1 à 1,2 équivalent molaire, par rapport à l'acide 2-céto-L-gulonique contenu dans la charge de fermentation, de l'amine à chaîne longue.

4. Procédé selon la revendication 2, caractérisé en ce qu'on extrait la charge de fermentation exempte ions calcium avec environ 2 à 6 parties en poids d'un alcanol en C₄ - C₈ par rapport à l'amine.

5. Procédé selon la revendication 1, caractérisé en ce qu'on utilise comme amine de la trioctylamine, de la tridodécylamine ou de la ditridécylamine.

6. Procédé selon la revendication 1, caractérisé en ce qu'on utilise un butanol comme alcanol en C₄ - C₈.

7. Procédé selon la revendication 2, caractérisé en ce qu'on retransforme par coupure produit d'addition obtenu acide 2-céto-L-gulonique/amine, en chauffant à une température comprise entre 60 et 120°C, en l'acide 2-céto-L-gulonique libre et l'amine utilisée.

8. Procédé selon la revendication 2, caractérisé en ce qu'on coupe le produit d'addition obtenu acide 2-céto-L-gulonique/amine par traitement avec 2 à 20 parties en poids d'eau, par rapport au produit d'addition, à des températures de 5 à 50°C, en l'acide 2-céto-L-gulonique libre et l'amine utilisée.

9. Procédé selon la revendication 2, caractérisé en ce qu'on transforme le produit d'addition obtenu acide 2-céto-L-gulonique/amine avec un hydroxyde alcalin ou un hydroxyde alcalino-terreux en sel correspondant de l'acide 2-céto-L-gulonique.

10. Procédé selon la revendication 2, caractérisé en ce qu'on transforme le produit d'addition obtenu acide 2-céto-L-gulonique/amine avec un alcanol en C₁ - C₅ contenant du gaz chlorhydrique, éventuellement dans un solvant inerte, en ester d'alcanol correspondant de l'acide 2-céto-gulonique.
